# EUROPÄISCHE PATENTSCHRIFT

(11) **EP 4 370 063 B1**
(45) Veröffentlichungstag und Bekanntmachung des Hinweises auf die Patenterteilung: **18.12.2024**
(21) Anmeldenummer: 22751292.8
(22) Anmeldetag: 12.07.2022
(51) Int. Cl.: A61F 2/16

(54) **INJEKTOR MIT EINER EINE MEHRZAHL AN ARMEN AUFWEISENDEN KOLBENSPITZE**
INJECTOR WITH A PISTON TIP HAVING A PLURALITY OF ARMS
INJECTEUR COMPORTANT UN EMBOUT DE PISTON DOTÉ D'UNE PLURALITÉ DE BRAS

(30) Priorität: 12.07.2021 DE 102021117965
(43) Veröffentlichungstag der Anmeldung: 22.05.2024
(73) Patentinhaber: Carl Zeiss Meditec AG, 07745 Jena (DE)
(72) Erfinder: MOEIN, Hadi, 73447 Oberkochen (DE)
(74) Vertreter: PATERIS Patentanwälte PartmbB
(86) Internationale Anmeldenummer: PCT/EP2022/069368
(87) Internationale Veröffentlichungsnummer: WO 2023/285410

(56) Entgegenhaltungen:
- EP-A1- 3 494 927
- FR-A1- 2 789 890
- US-A1- 2007 005 135

## Beschreibung

Die Erfindung betrifft einen Injektor für ein Einführen einer Intraokularlinse in den Kapselsack eines Auges. US 2007/005135 A1 offenbart einen Stößel zum Einsetzen einer Intraokularlinse, wobei der Stößel eine weiche Spitze hat. FR 2 789 890 A1 offenbart eine Spritze zur Implantation einer weichen Intraokularlinse mit einem einteiligen Schaft, der durch einen konischen Abschnitt mit einer zylindrischen Spitze verbunden ist. EP 3 494 927 A1 offenbart eine männliche Form, die als Anschlusselement für einen Injektor ausgebildet ist. Bei einer Kataraktbehandlung eines Auges wird herkömmlich nur ein kleiner Schnitt in die Hornhaut des Auges eingebracht, der so groß ist, dass eine Spitze eines Injektors durch den Schnitt in das Auge eingeführt werden kann. Nachdem der Schnitt in die Hornhaut eingebracht wurde, wird die Linse des Auges beispielsweise mittels Phakoemulsifikation zerkleinert und anschließend aus dem Kapselsack des Auges abgesaugt. Danach wird eine Intraokularlinse mittels des Injektors in das Auge eingesetzt. Die Intraokularlinse wird in dem Injektor gefaltet, so dass sie durch die Spitze passt. Die Spitze wird durch den Schnitt in den Kapselsack eingeführt und die gefaltete Intraokularlinse wird mittels eines Kolbens des Injektors durch die Spitze in den Kapselsack geschoben, in dem die Intraokularlinse sich entfaltet und somit die ursprüngliche Linse ersetzt.

Um den Schnitt in der Hornhaut möglichst klein ausführen zu können, ist es erforderlich, dass die Spitze des Injektors möglichst klein ausgeführt ist. Dies führt dazu, dass die Intraokularlinse und der Kolben stark gequetscht werden, wenn der Kolben die Intraokularlinse in die Spitze schiebt, wodurch eine hohe mechanische Belastung der Intraokularlinse und des Kolbens resultiert und im Extremfall eine Beschädigung der Intraokularlinse und/oder des Kolbens eintreten kann. Das starke Quetschen der Intraokularlinse und des Kolbens führt ebenfalls zu einer hohen mechanischen Belastung der Spitze, wobei die hohe mechanische Belastung zu einer Beschädigung der Spitze führen kann. Beispielsweise können Teile der Spitze irreversibel nach außen verformt werden, was im Extremfall dazu führen kann, dass die Spitze eine Erweiterung des Schnitts und/oder eine Beschädigung des Auges verursachen kann, wenn die Spitze aus dem Auge herausgezogen wird.

Aufgabe der Erfindung ist es daher, einen Injektor für ein Einführen einer Intraokularlinse in den Kapselsack eines Auges zu schaffen, wobei eine Spitze des Injektors klein ausgeführt werden kann, ohne dass die Gefahr einer Beschädigung der Spitze auftritt.

Der erfindungsgemäße Injektor für ein Einführen einer Intraokularlinse in den Kapselsack eines Auges weist eine Injektorspitze, die eine Öffnung begrenzt, und einen Kolben auf, der längsverlagerbar entlang einer Längsachse des Injektors in dem Injektor gelagert ist und eine Kolbenspitze aufweist, die aus einem Werkstoff mit einer Härte in einem Bereich von 20 bis 80 Shore A ausgebildet ist und eingerichtet ist, die Intraokularlinse via die Öffnung aus dem Injektor zu verlagern, wobei die Kolbenspitze einen zentralen Bereich und eine Mehrzahl an Armen aufweist, die an dem zentralen Bereich befestigt sind und in Umfangsrichtung bezüglich der Längsachse nebeneinander angeordnet sind, wobei die Kolbenspitze einen entspannten Zustand hat, in dem die Arme jeweils einen inneren Bereich und einen äußeren Bereich aufweisen, der weiter entfernt als der innere Bereich von dem zentralen Bereich angeordnet ist und in der Umfangsrichtung eine längere Erstreckung als der innere Bereich hat. Indem die Kolbenspitze aus dem Werkstoff mit der Härte in dem Bereich von 10 bis 80 Shore A ausgebildet ist und die Mehrzahl der Arme aufweist, kann sie sich verformen, wenn sie in die Injektorspitze gelangt. Dadurch, dass der innere Bereich die kürzere Erstreckung in der Umfangsrichtung als der äußere Bereich hat, wird der innere Bereich eher als der äußere Bereich verformen. Dadurch kann der äußere Bereich sich in der Umfangsrichtung verlagern und sich sogar in Richtung zu dem zentralen Bereich hin verlagern. Damit kann die Kolbenspitze ausgehend von ihrem entspannten Zustand stark gequetscht werden, ohne dass übermäßige mechanische Spannungen in der Kolbenspitze entstehen, die zu einer Beschädigung der Injektorspitze führen können. Dadurch ist es möglich, dass die Injektorspitze eher klein ausgeführt wird, ohne dass eine Gefahr einer Beschädigung der Injektorspitze auftritt.

Es ist bevorzugt, dass die Härte in einem Bereich von 20 bis 80 Shore A und insbesondere von 20 bis 50 Shore A liegt.

Es ist erfindungsgemäß, dass die Kolbenspitze eine Stirnfläche aufweist, die der Öffnung zugewandt angeordnet ist und die in dem entspannten Zustand einen Mittelpunkt aufweist, durch den die Längsachse verläuft, wobei die Arme in dem entspannten Zustand jeweils einen ersten Punkt aufweisen, der derjenige Punkt ist, der am weitesten von der Längsachse entfernt ist, wobei die Kolbenspitze einen teilweise vorgespannten Zustand hat, in dem die Arme durch eine Kraft, die in Radialrichtung bezüglich der Längsachse von außen auf die Arme wirkt, vorgespannt sind und in Richtung zu der Längsachse hin verlagert sind und in dem die Arme jeweils einen zweiten Punkt aufweisen, der derjenige Punkt ist, der am weitesten von der Längsachse entfernt ist, wobei der zweite Punkt näher an der Längsachse als der erste Punkt angeordnet ist. Die Kraft, die in der Radialrichtung von außen auf die Arme wirkt, wird erzeugt, wenn die Kolbenspitze eine Wand des Injektors, insbesondere die Wand der Injektorspitze, kontaktiert. Dadurch, dass die Stirnfläche der Öffnung zugewandt angeordnet ist, kontaktiert die Stirnfläche die Intraokularlinse, wenn der Kolben in Richtung zu der Öffnung hin längsverlagert wird. Der Mittelpunkt ist der Schwerpunkt der Stirnfläche und ist in dem zentralen Bereich angeordnet. Es ist besonders bevorzugt, dass der zweifache Abstand des ersten Punkts zu der Längsachse länger ist als eine maximale Erstreckung der Öffnung, wobei die maximale Erstreckung der Öffnung bestimmt ist, indem die Öffnung auf eine Ebene projiziert ist, deren Normale parallel zu der Längsachse orientiert ist, und die maximale Erstreckung der Öffnung als eine maximale Erstreckung der auf die Ebene projizierten Öffnung bestimmt ist. Dadurch hat die Injektorspitze in dem entspannten Zustand einen längeren Durchmesser als die maximale Erstreckung der Öffnung.

Die Kolbenspitze hat bevorzugt einen vollständig vorgespannten Zustand, den die Kolbenspitze annimmt, nachdem die Kolbenspitze ausgehend von dem entspannten Zustand den teilweise vorgespannten Zustand durchlaufen hat, und in dem einer der Arme an den zentralen Bereich und/oder an einen anderen Arm angestoßen ist, der in Umfangsrichtung benachbart zu dem einen Arm angeordnet ist. Es ist auch denkbar, dass in dem vollständigen vorgespannten Zustand eine Mehrzahl oder alle der Arme an den zentralen Bereich und/oder an den jeweils benachbarten Arm angestoßen sind.

Es ist bevorzugt, dass der Injektor eine Injektorspitze aufweist, die die Öffnung bildet und die einen Innenraum begrenzt, der sich in Richtung zu der Öffnung hin verjüngt. Dadurch kann die Injektorspitze allmählich von dem entspannten Zustand, via den teilweise vorgespannten Zustand bis in den vollständig vorgespannten Zustand gebracht werden.

Der Kolben weist bevorzugt einen Kolbenschaft auf, an dem die Kolbenspitze angeordnet ist und der aus einem Werkstoff mit einer Härte von mindestens 20 Shore A ausgebildet ist. Dadurch wird vorteilhaft erreicht, dass lediglich die Kolbenspitze des Kolbens verformt wird. Somit kann eine übermäßige Verkürzung des Kolbens in Richtung der Längsachse unterbunden werden.

Der Kolben kann beispielsweise mittels eines Spritzgussverfahrens hergestellt werden. Insbesondere können die Kolbenspitze und der Kolbenschaft mit zwei unterschiedlichen Injektionsschritten hergestellt werden. Ein solches Verfahren ist beispielsweise unter dem Namen 2K Spritzgussverfahren bekannt. Der Werkstoff der Kolbenspitze kann verschieden vom dem Werkstoff des Kolbenschafts sein.

Die Arme sind bevorzugt baugleich geformt. Es ist zudem bevorzugt, dass in dem entspannten Zustand alle die Arme in Umfangsrichtung in gleichen Abständen zu den jeweils benachbarten Armen angeordnet sind. Es sind bevorzugt von 3 bis 5 der Arme vorgesehen.

Es ist bevorzugt, dass in dem entspannten Zustand jeder der Arme eine Außenlinie hat, dessen Punkte in jeder axialen Position derjenige Punkt des jeweiligen Arms ist, der am weitesten von der Injektorachse entfernt ist, wobei der Abstand der Außenlinie zu der Längsachse zumindest in einem Bereich entlang der Längsachse mit zunehmenden Abstand von der Öffnung kürzer wird. Dadurch kann die Injektorspitze leichter verformt werden als in dem Fall, dass die Außenlinie entlang der gesamten Injektorspitze den gleichen Abstand zu der Längsachse hat. Es ist besonders bevorzugt, dass die Außenlinie ein Minimum hat, das derjenige Punkt der Außenlinie ist, der den kürzesten Abstand zu der Längsachse hat, wobei das Minimum einen kürzeren Abstand zu der Längsachse hat als jeder Punkt auf der Außenseite des Kolbenschafts an seinem der Kolbenspitze zugewandten Längsende. Dabei kann die Kolbenspitze für jeden Arm eine Verstärkungsrippe aufweisen, die in einem axialen Bereich des Kolbens angeordnet ist, in dem auch das Minimum angeordnet ist, und die zwei benachbarte der Arme in der Umfangsrichtung abstützt.

Die Arme weisen bevorzugt keine Aussparungen in ihrem Inneren auf. Dadurch können die Arme besonders einfach hergestellt werden.

Es ist bevorzugt, dass die Arme in dem entspannten Zustand in der Umfangsrichtung gekrümmt sind. Dadurch können die Arme sich besonders einfach in die Umfangsrichtung verlagern, wenn eine Kraft in Radialrichtung bezüglich der Längsachse von außen auf die Arme wirkt. Besonders bevorzugt sind alle die Arme in die gleiche Richtung gekrümmt.

Im Folgenden wird anhand der beigefügten schematischen Zeichnungen die Erfindung näher erläutert. Es zeigen
Figuren 1 bis 4 eine erste Ausführungsform einer Injektorspitze zu vier verschiedenen Zeitpunkten,
Figuren 5 und 6 eine zweite Ausführungsform der Injektorspitze zu zwei verschiedenen Zeitpunkten,
Figur 7 eine Ansicht von vorne auf eine erste Ausführungsform eines Kolbens,
Figur 8 eine Ansicht seitlich von vorne auf die erste Ausführungsform gemäß Figur 7 und
Figur 9 eine seitliche Ansicht auf einen Injektor mit der ersten Ausführungsform gemäß Figuren 7 und 8.
Figur 10 eine seitliche Ansicht auf eine zweite Ausführungsform des Kolbens.

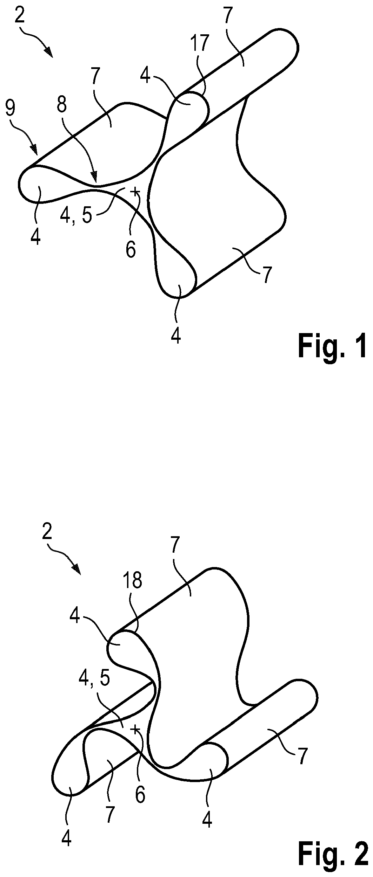

Wie es aus Figuren 1 bis 9 ersichtlich ist, weist ein Injektor 10 für ein Einführen einer Intraokularlinse in den Kapselsack eines Auges eine Injektorspitze 14, die eine Öffnung 15 begrenzt, und einen Kolben 1 auf. Der Kolben 1 ist längsverlagerbar entlang einer Längsachse 11 des Injektors 10 in dem Injektor 10 gelagert und weist eine Kolbenspitze 2 auf, die aus einem Werkstoff mit einer Härte in einem Bereich von 10 bis 80 Shore A ausgebildet ist und eingerichtet ist, die Intraokularlinse via die Öffnung 15 aus dem Injektor 10 zu verlagern. Die Kolbenspitze 2 weist einen zentralen Bereich 5 und eine Mehrzahl an Armen 7 auf, die an dem zentralen Bereich 5 befestigt sind und in Umfangsrichtung 13 bezüglich der Längsachse 11 nebeneinander angeordnet sind. Die Kolbenspitze 2 hat einen entspannten Zustand, in dem die Arme 7 jeweils einen inneren Bereich 8 und einen äußeren Bereich 9 aufweisen, der weiter entfernt als der innere Bereich 8 von dem zentralen Bereich 5 angeordnet ist und in der Umfangsrichtung 13 eine längere Erstreckung als der innere Bereich 8 hat.

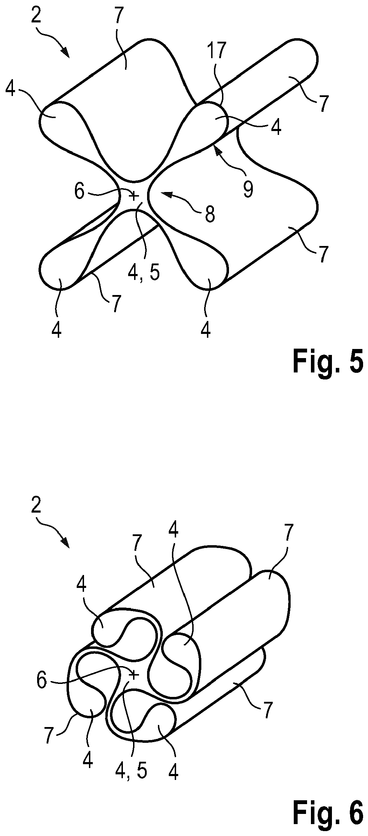

Figuren 1 bis 9 zeigen zudem, dass alle die Arme 7 baugleich geformt sein können. Außerdem können in dem entspannten Zustand alle die Arme 7 in Umfangsrichtung 13 beabstandet zueinander angeordnet sein und insbesondere in gleichen Abständen zu den jeweils benachbarten Armen 7 angeordnet sein. Figuren 1 bis 4 und 7 bis 9 zeigen, dass beispielsweise drei der Arme 7 vorgesehen sein können. Figuren 5 und 6 zeigen, dass beispielsweise vier der Arme vorgesehen sein können. Es sind doch jedoch auch andere Anzahlen der Arme 7 denkbar, wie beispielsweise fünf, sechs oder sieben der Arme 7. Ferner ist denkbar, dass die Arme 7 keine Aussparungen in ihrem Inneren aufweisen.

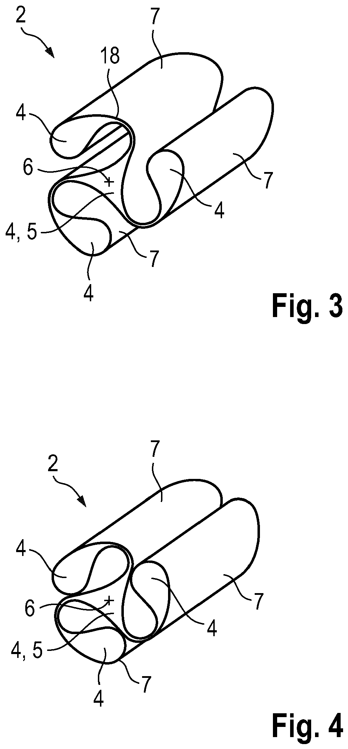

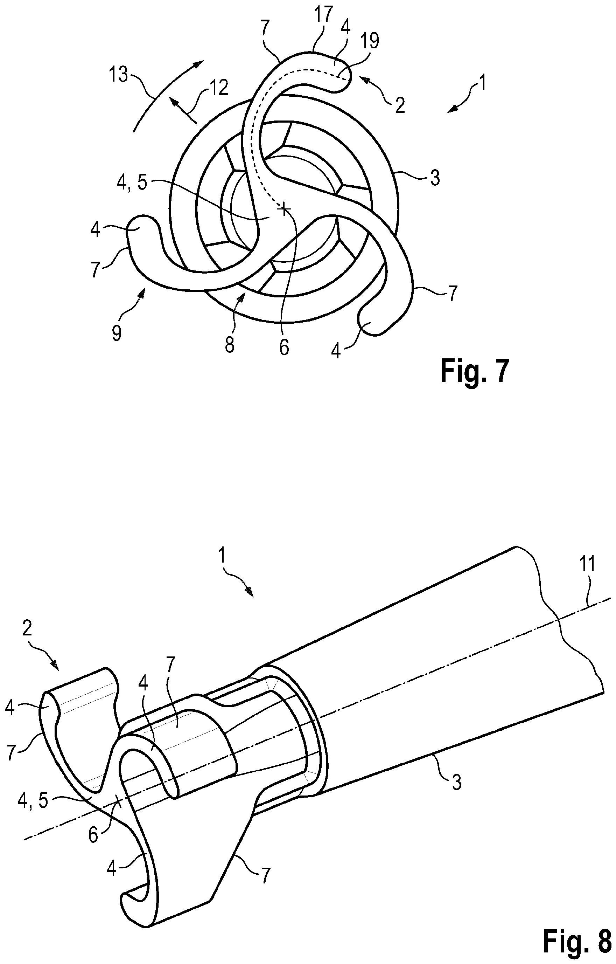

Figuren 1, 5 und 7 bis 9 zeigen die Kolbenspitze 2 in dem entspannten Zustand. Wie es aus Figuren 1 bis 8 ersichtlich ist, kann die Kolbenspitze 2 eine Stirnfläche 4 aufweisen, die der Öffnung 15 zugewandt angeordnet ist und die in dem entspannten Zustand einen Mittelpunkt 6 aufweist, durch den die Längsachse 11 verläuft (vergleiche Figur 8), wobei die Arme 7 in dem entspannten Zustand jeweils einen ersten Punkt 17 aufweisen, der derjenige Punkt ist, der am weitesten von der Längsachse 11 entfernt ist, wobei die Kolbenspitze 2 einen teilweise vorgespannten Zustand haben kann, der in Figuren 2 und 3 dargestellt ist und in dem die Arme 7 durch eine Kraft, die in Radialrichtung 12 bezüglich der Längsachse 11 von außen auf die Arme 7 wirkt, vorgespannt sind und in Richtung zu der Längsachse 11 hin verlagert sind und in dem die Arme 7 jeweils einen zweiten Punkt 18 aufweisen, der derjenige Punkt ist, der am weitesten von der Längsachse 11 entfernt ist, wobei der zweite Punkt 18 in dem teilweise vorgespannten Zustand näher an der Längsachse 11 als der erste Punkt 17 in dem entspannten Zustand angeordnet ist. Der Mittelpunkt 6 kann dabei der Schwerpunkt der Stirnfläche 4 in dem entspannten Zustand sein. In Figur 3 ist die Kolbenspitze 2 stärker vorgespannt als in Figur 2, wodurch der zweite Punkt 18 in Figur 3 näher an der Längsachse 11 als in Figur 2 angeordnet ist. Es ist in Figuren 2 und 3 erkennbar, dass die Arme 7 insbesondere in dem inneren Bereich 8 im Vergleich zu dem entspannten Zustand (siehe Figur 1) verformt sind, so dass alle die Arme 7 in derselben Umfangsrichtung 13 verbogen sind.

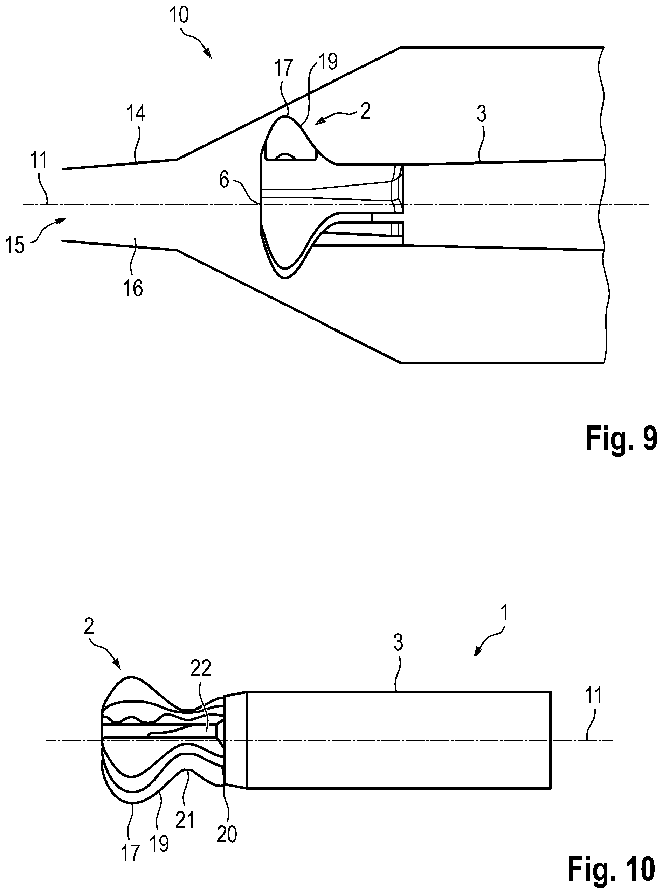

Aus Figur 9 ist ersichtlich, dass der zweifache Abstand des ersten Punkts 17 zu der Längsachse 11 länger als eine maximale Erstreckung der Öffnung 15 sein kann, wobei die maximale Erstreckung der Öffnung 15 bestimmt ist, indem die Öffnung 15 auf eine Ebene projiziert ist, deren Normale parallel zu der Längsachse 11 orientiert ist, und die maximale Erstreckung der Öffnung 15 als eine maximale Erstreckung der auf die Ebene projizierten Öffnung 15 bestimmt ist.

Figur 4 zeigt, dass die Kolbenspitze 2 einen vollständig vorgespannten Zustand haben kann, den die Kolbenspitze 2 annimmt, nachdem die Kolbenspitze 2 ausgehend von dem entspannten Zustand den teilweise vorgespannten Zustand durchlaufen hat, und in dem einer der Arme 7 an den einen anderen Arm 7 angestoßen ist, der in Umfangsrichtung 13 benachbart zu dem einen Arm 7 angeordnet ist. Alternativ oder zusätzlich ist denkbar, dass der eine Arm 7 in dem vollständig vorgespannten Zustand an den zentralen Bereich 5 angestoßen ist. Zudem ist denkbar, dass alle die Arme 7 an einen benachbarten Arm 7 und/oder den zentralen Bereich 5 angestoßen sind.

Figuren 7 bis 9 zeigen, dass der Kolben 1 einen Kolbenschaft 3 aufweisen kann, an dem die Kolbenspitze 2 angeordnet ist und der aus einem Werkstoff mit einer Härte von mindestens 20 Shore A ausgebildet ist. Aus Figur 9 ist zudem ersichtlich, dass der vollständige Kolbenschaft 3 in einem in Radialrichtung 12 bezüglich der Längsachse 11 sich erstreckenden Bereich angeordnet sein kann, der weniger weit entfernt von der Längsachse 11 als der erste Punkt 17 angeordnet ist.

Aus Figuren 9 und 10 ist ersichtlich, dass in dem entspannten Zustand jeder der Arme 7 eine Außenlinie 19 haben kann, dessen Punkte in jeder axialen Position derjenige Punkt des jeweiligen Arms 7 ist, der am weitesten von der Injektorachse 11 entfernt ist, wobei der Abstand der Außenlinie 19 zu der Längsachse 11 zumindest in einem Bereich entlang der Längsachse 11 mit zunehmenden Abstand von der Öffnung 15 kürzer wird. Zudem zeigt Figur 9, dass die Injektorspitze 14 einen Innenraum 16 begrenzen kann, der sich in Richtung zu der Öffnung 15 hin verjüngt.

Figur 10 zeigt, dass die Außenlinie 19 ein Minimum 21 hat, das derjenige Punkt der Außenlinie 19 ist, der den kürzesten Abstand zu der Längsachse 11 hat, wobei das Minimum 21 einen kürzeren Abstand zu der Längsachse 11 hat als jeder Punkt auf der Außenseite des Kolbenschafts 3 an seinem der Kolbenspitze 2 zugewandten Längsende 20. Dabei kann die Kolbenspitze 3 für jeden Arm 7 eine Verstärkungsrippe 22 aufweisen, die in einem axialen Bereich des Kolbens 1 angeordnet ist, in dem auch das Minimum 21 angeordnet ist, und die zwei benachbarte der Arme 7 in der Umfangsrichtung 13 abstützt.

Figuren 7 und 8 zeigen, dass die Arme 7 in dem entspannten Zustand in der Umfangsrichtung 13 gekrümmt sein können. Insbesondere kann eine Mittellinie 19, die auf der Stirnfläche 4 angeordnet ist sowie in der Mitte des Arms 7 und entlang der gesamten Länge des Arms 7 verläuft, gekrümmt ausgeführt sein.

### Bezugszeichenliste

1 Kolben
2 Kolbenspitze
3 Kolbenschaft
4 Stirnfläche
5 zentraler Bereich
6 Mittelpunkt
7 Arm
8 innerer Bereich
9 äußerer Bereich
10 Injektor
11 Längsachse
12 Radialrichtung
13 Umfangsrichtung
14 Injektorspitze
15 Öffnung
16 Innenraum
17 erster Punkt
18 zweiter Punkt
19 Außenlinie
20 Längsende des Kolbenschafts
21 Minimum
22 Verstärkungsrippe

## Patentansprüche

1. Injektor für ein Einführen einer Intraokularlinse in den Kapselsack eines Auges, mit einer Injektorspitze (14), die eine Öffnung (15) begrenzt, und einem Kolben (1), der längsverlagerbar entlang einer Längsachse (11) des Injektors (10) in dem Injektor (10) gelagert ist und eine Kolbenspitze (2) aufweist, die aus einem Werkstoff mit einer Härte in einem Bereich von 10 bis 80 Shore A ausgebildet ist und eingerichtet ist, die Intraokularlinse via die Öffnung (15) aus dem Injektor (10) zu verlagern, **dadurch gekennzeichnet, dass** die Kolbenspitze (2) einen zentralen Bereich (5) und eine Mehrzahl an Armen (7) aufweist, die an dem zentralen Bereich (5) befestigt sind und in Umfangsrichtung (13) bezüglich der Längsachse (11) nebeneinander angeordnet sind, wobei die Kolbenspitze (2) einen entspannten Zustand hat, in dem die Arme (7) jeweils einen inneren Bereich (8) und einen äußeren Bereich (9) aufweisen, der weiter entfernt als der innere Bereich (8) von dem zentralen Bereich (5) angeordnet ist und in der Umfangsrichtung (13) eine längere Erstreckung als der innere Bereich (8) hat, wobei die Kolbenspitze (2) eine Stirnfläche (4) aufweist, die der Öffnung (15) zugewandt angeordnet ist und die in dem entspannten Zustand einen Mittelpunkt (6) aufweist, durch den die Längsachse (11) verläuft, wobei die Arme (7) in dem entspannten Zustand jeweils einen ersten Punkt (17) aufweisen, der derjenige Punkt ist, der am weitesten von der Längsachse (11) entfernt ist, wobei die Kolbenspitze (2) einen teilweise vorgespannten Zustand hat, in dem die Arme (7) durch eine Kraft, die in Radialrichtung (12) bezüglich der Längsachse (11) von außen auf die Arme (7) wirkt, vorgespannt sind und in Richtung zu der Längsachse (11) hin verlagert sind und in dem die Arme (7) jeweils einen zweiten Punkt (18) aufweisen, der derjenige Punkt ist, der am weitesten von der Längsachse (11) entfernt ist, wobei der zweite Punkt (18) näher an der Längsachse (11) als der erste Punkt (17) angeordnet ist.

2. Injektor gemäß Anspruch 1, wobei der zweifache Abstand des ersten Punkts (17) zu der Längsachse (11) länger ist als eine maximale Erstreckung der Öffnung (15), wobei die maximale Erstreckung der Öffnung (15) bestimmt ist, indem die Öffnung (15) auf eine Ebene projiziert ist, deren Normale parallel zu der Längsachse (11) orientiert ist, und die maximale Erstreckung der Öffnung (15) als eine maximale Erstreckung der auf die Ebene projizierten Öffnung (15) bestimmt ist.

3. Injektor gemäß Anspruch 1 oder 2, wobei die Kolbenspitze (2) einen vollständig vorgespannten Zustand hat, den die Kolbenspitze (2) annimmt, nachdem die Kolbenspitze (2) ausgehend von dem entspannten Zustand den teilweise vorgespannten Zustand durchlaufen hat, und in dem einer der Arme (7) an den zentralen Bereich (5) und/oder an einen anderen Arm (7) angestoßen ist, der in Umfangsrichtung (13) benachbart zu dem einen Arm (7) angeordnet ist.

4. Injektor gemäß einem der Ansprüche 1 bis 3, wobei die Injektorspitze (14) einen Innenraum (16) begrenzt, der sich in Richtung zu der Öffnung (15) hin verjüngt.

5. Injektor gemäß einem der Ansprüche 1 bis 4, wobei der Kolben (1) einen Kolbenschaft (3) aufweist, an dem die Kolbenspitze (2) angeordnet ist und der aus einem Werkstoff mit einer Härte von mindestens 20 Shore A ausgebildet ist.

6. Injektor gemäß einem der Ansprüche 1 bis 5, wobei die Arme (7) baugleich geformt sind.

7. Injektor gemäß einem der Ansprüche 1 bis 6, wobei in dem entspannten Zustand jeder der Arme (7) eine Außenlinie hat, dessen Punkte in jeder axialen Position derjenige Punkt des jeweiligen Arms (7) ist, der am weitesten von der Injektorachse (11) entfernt ist, wobei der Abstand der Außenlinie zu der Längsachse (11) zumindest in einem Bereich entlang der Längsachse (11) mit zunehmenden Abstand von der Öffnung (15) kürzer wird.

8. Injektor gemäß einem der Ansprüche 1 bis 7, wobei die Arme (7) keine Aussparungen in ihrem Inneren aufweisen.

9. Injektor gemäß einem der Ansprüche 1 bis 8, wobei die Arme (7) in dem entspannten Zustand in der Umfangsrichtung (13) gekrümmt sind.

## Claims

1. Injector for inserting an intraocular lens into the capsular bag of an eye, having an injector tip (14), which delimits an opening (15), and a plunger (1), which is mounted in the injector (10) in such a way as to be longitudinally displaceable along a longitudinal axis (11) of the injector (10) and has a plunger tip (2) which is made of a material with a hardness in a range of 10 to 80 Shore A and is configured to move the intraocular lens out of the injector (10) via the opening (15), **characterized in that** the plunger tip (2) has a central region (5) and a plurality of arms (7), which are attached to the central region (5) and are arranged next to one another in the circumferential direction (13) with respect to the longitudinal axis (11), wherein the plunger tip (2) has a relaxed state in which the arms (7) each have an inner region (8) and an outer region (9), the latter being arranged further than the inner region (8) from the central region (5) and having a longer extent than the inner region (8) in the circumferential direction (13), wherein the plunger tip (2) has a front face (4), which is arranged facing the opening (15) and which, in the relaxed state, has a centre point (6) through which the longitudinal axis (11) runs, wherein the arms (7), in the relaxed state, each have a first point (17), which is the point furthest from the longitudinal axis (11), wherein the plunger tip (2) has a partially pretensioned state in which the arms (7) are pretensioned by a force acting on the arms (7) from the outside in a radial direction (12) with respect to the longitudinal axis (11) and are moved towards the longitudinal axis (11), and in which the arms (7) each have a second point (18), which is the point furthest from the longitudinal axis (11), wherein the second point (18) is arranged closer to the longitudinal axis (11) than the first point (17).

2. Injector according to Claim 1, wherein twice the distance from the first point (17) to the longitudinal axis (11) is longer than a maximum extent of the opening (15), wherein the maximum extent of the opening (15) is defined by the opening (15) being projected onto a plane whose normal is oriented parallel to the longitudinal axis (11), and the maximum extent of the opening (15) is defined as a maximum extent of the opening (15) projected onto the plane.

3. Injector according to Claim 1 or 2, wherein the plunger tip (2) has a fully pretensioned state that the plunger tip (2) assumes after the plunger tip (2), starting from the relaxed state, has passed through the partially pretensioned state, and in which one of the arms (7) abuts the central region (5) and/or another arm (7), which is arranged adjacent to the one arm (7) in the circumferential direction (13).

4. Injector according to any one of Claims 1 to 3, wherein the injector tip (14) delimits an interior space (16), which tapers in the direction of the opening (15).

5. Injector according to any one of Claims 1 to 4, wherein the plunger (1) has a plunger shaft (3), on which the plunger tip (2) is arranged and which is made of a material with a hardness of at least 20 Shore A.

6. Injector according to any one of Claims 1 to 5, wherein the arms (7) are shaped identically.

7. Injector according to any one of Claims 1 to 6, wherein, in the relaxed state, each of the arms (7) has an outer line, the points of which in each axial position are the point of the respective arm (7) furthest from the injector axis (11), wherein the distance of the outer line from the longitudinal axis (11), at least in one region along the longitudinal axis (11), becomes shorter as the distance from the opening (15) increases.

8. Injector according to any one of Claims 1 to 7, wherein the arms (7) have no recesses in their interior.

9. Injector according to any one of Claims 1 to 8, wherein the arms (7) are curved in the circumferential direction (13) in the relaxed state.

## Revendications

1. Injecteur pour une introduction d'une lentille intraoculaire dans le sac capsulaire d'un oeil, avec une pointe d'injecteur (14) qui délimite une ouverture (15), et un piston (1) qui est monté dans l'injecteur (10) de manière à pouvoir se déplacer longitudinalement le long d'un axe longitudinal (11) de l'injecteur (10) et qui présente une pointe de piston (2) qui est réalisée en un matériau avec une dureté dans une plage de 10 à 80 Shore A et qui est adaptée pour déplacer la lentille intraoculaire hors de l'injecteur (10) via l'ouverture (15), **caractérisé en ce que** la pointe de piston (2) présente une zone centrale (5) et une pluralité de bras (7) fixés à la zone centrale (5) et agencés côte à côte dans la direction circonférentielle (13) par rapport à l'axe longitudinal (11), la pointe de piston (2) ayant un état relâché dans lequel les bras (7) présentent chacun une zone intérieure (8) et une zone extérieure (9), qui est agencée plus loin que la zone intérieure (8) de la zone centrale (5) et qui a une extension plus longue dans la direction circonférentielle (13) que la zone intérieure (8), la pointe de piston (2) présentant une face frontale (4), qui est agencée en face de l'ouverture (15) et qui, dans l'état relâché, présente un centre (6) par lequel passe l'axe longitudinal (11), les bras (7) présentant chacun, dans l'état relâché, un premier point (17) qui est le point le plus éloigné de l'axe longitudinal (11), la pointe de piston (2) ayant un état partiellement précontraint dans lequel les bras (7) sont précontraints par une force agissant sur les bras (7) dans la direction radiale (12) par rapport à l'axe longitudinal (11) depuis l'extérieur, et sont déplacés en direction de l'axe longitudinal (11), et les bras (7) présentant chacun un deuxième point (18) qui est le point le plus éloigné de l'axe longitudinal (11), le deuxième point (18) étant agencé plus près de l'axe longitudinal (11) que le premier point (17).

2. Injecteur selon la revendication 1, le double de la distance du premier point (17) à l'axe longitudinal (11) étant plus long qu'une extension maximale de l'ouverture (15), l'extension maximale de l'ouverture (15) étant déterminée en projetant l'ouverture (15) sur un plan dont la normale est orientée parallèlement à l'axe longitudinal (11), et l'extension maximale de l'ouverture (15) étant déterminée comme une extension maximale de l'ouverture (15) projetée sur le plan.

3. Injecteur selon la revendication 1 ou 2, la pointe de piston (2) ayant un état complètement précontraint que la pointe de piston (2) adopte après que la pointe de piston (2) est passée par l'état partiellement précontraint à partir de l'état relâché, et dans lequel l'un des bras (7) est en butée contre la zone centrale (5) et/ou contre un autre bras (7) qui est agencé de manière adjacente au bras (7) dans la direction circonférentielle (13).

4. Injecteur selon l'une quelconque des revendications 1 à 3, la pointe d'injecteur (14) délimitant un espace intérieur (16) qui se rétrécit en direction de l'ouverture (15).

5. Injecteur selon l'une quelconque des revendications 1 à 4, le piston (1) présentant une tige de piston (3) sur laquelle est agencée la pointe de piston (2) et qui est réalisée en un matériau ayant une dureté d'au moins 20 Shore A.

6. Injecteur selon l'une quelconque des revendications 1 à 5, les bras (7) étant de forme identique.

7. Injecteur selon l'une quelconque des revendications 1 à 6, dans l'état relâché, chacun des bras (7) ayant une ligne extérieure dont les points, dans chaque position axiale, sont le point du bras respectif (7) qui est le plus éloigné de l'axe d'injecteur (11), la distance de la ligne extérieure à l'axe longitudinal (11) devenant plus courte au moins dans une zone le long de l'axe longitudinal (11) à mesure que la distance à l'ouverture (15) augmente.

8. Injecteur selon l'une quelconque des revendications 1 à 7, les bras (7) ne présentant pas d'évidements à l'intérieur de ceux-ci.

9. Injecteur selon l'une quelconque des revendications 1 à 8, les bras (7) étant incurvés dans la direction circonférentielle (13) à l'état relâché.
